(19) Europäisches Patentamt

European Patent Office

Office européen des brevets

(11) **EP 0 770 682 A2**

(12) **EUROPEAN PATENT APPLICATION**

(43) Date of publication:
02.05.1997 Bulletin 1997/18

(51) Int. Cl.$^6$: **C12N 15/82**, A01H 5/00

(21) Application number: 96400089.7

(22) Date of filing: 15.01.1996

(84) Designated Contracting States:
BE CH DE FR GB LI NL

(30) Priority: 11.10.1995 KR 9534790

(71) Applicant: JINRO LIMITED
Seoul (KR)

(72) Inventors:
• Yun, Young-Chae
Jangan-Ku, Suwon, Kyonggi-Do (KR)
• Moon, Young-Ho
Kuri, Kyonggi-Do (KR)
• Choi, Jin-Nam
Kwonsun-Ku, Suwon, Kyonggi-Do (KR)

• Choi, Kyu-Whan
Hwahyun-Myun, Pochun-Kun, Kyonggi-Do (KR)
• Kim, Chul-Hwan
Seocho-Ku, Seoul (KR)
• Kim, Man-Keun
Youngdeungpo-Ku, Seoul (KR)
• Guh, Ja-Ock
Buk-Ku, Kwangju (KR)
• Jeon, Hong-Seob
Yongin-Kun, Kyonggi-Do (KR)

(74) Representative: Hirsch, Marc-Roger et al
Cabinet Hirsch
34 rue de Bassano
75008 Paris (FR)

(54) **Herbicide-tolerant transgenic plant**

(57) The present invention provides a herbicide-tolerant transgenic plant producing Protox(protoporphyrinogen oxidase) which gives the plant a resistance against DPE(diphenylether) -derived herbicide, and a process for preparing the same. The process for preparing a herbicide-tolerant transgenic plant comprises the step of culturing plant cells transformed with a recombinant expression vector containing Protox gene. The transgenic plant of the invention possesses a resistance against DPE-derived herbicide with the help of recombinant Protox. Accordingly, the process for preparing the herbicide-tolerant transgenic plant of the invention, can be applied for the preparation of a variety of plants which are protected from the phytotoxicity of DPE-derived herbicide by way of introducing proper recombinant expression vectors containing Protox gene.

FIGURE 3(A)

SEQUENCE LISTING

(1) GENERAL INFORMATION:

    (i) APPLICANT:
        (A) NAME: JINRO LIMITED
        (B) STREET: 1448-3, Seocho-Dong, Seocho-Ku
        (C) CITY: Seoul
        (D) STATE: not applicable
        (E) COUNTRY: Korea
        (F) POSTAL CODE (ZIP): 137-070

    (ii) TITLE OF INVENTION: HERBICIDE-TOLERANT TRANSGENIC PLANT

    (iii) NUMBER OF SEQUENCES: 3

    (iv) COMPUTER READABLE FORM:
        (A) MEDIUM TYPE: Floppy disk
        (B) COMPUTER: IBM PC compatible
        (C) OPERATING SYSTEM: PC-DOS/MS-DOS
        (D) SOFTWARE: PatentIn Release #1.0, Version #1.30(EPO)

(2) INFORMATION FOR SEQ ID NO: 1:

    (i) SEQUENCE CHARACTERISTICS:
        (A) LENGTH: 33 base pairs
        (B) TYPE: nucleic acid
        (C) STRANDEDNESS: single
        (D) TOPOLOGY: linear

    (ii) MOLECULE TYPE: cDNA to mRNA

    (vii) IMMEDIATE SOURCE:
        (B) CLONE: N-TERMINAL PRIMER

    (xi) SEQUENCE DESCRIPTION: SEQ ID NO: 1:

        GCCGAAGCTT GGATCCATGA GTGACCGCAA AAA        33

(2) INFORMATION FOR SEQ ID NO: 2:

    (i) SEQUENCE CHARACTERISTICS:
        (A) LENGTH: 28 base pairs
        (B) TYPE: nucleic acid
        (C) STRANDEDNESS: single
        (D) TOPOLOGY: linear

    (ii) MOLECULE TYPE: cDNA to mRNA

    (vii) IMMEDIATE SOURCE:
        (B) CLONE: C-TERMINAL PRIMER

    (xi) SEQUENCE DESCRIPTION: SEQ ID NO: 2:

        GCCGTCTAGA GTTTTAGCTG AATAAAAT        28

```
(2)  INFORMATION FOR SEQ ID NO: 3:

        (i)  SEQUENCE CHARACTERISTICS:
              (A)  LENGTH: 33 base pairs
              (B)  TYPE: nucleic acid
              (C)  STRANDEDNESS: single
              (D)  TOPOLOGY: linear

       (ii)  MOLECULE TYPE: cDNA to mRNA

      (vii)  IMMEDIATE SOURCE:
              (B)  CLONE: C-TERMINAL PRIMER

       (xi)  SEQUENCE DESCRIPTION: SEQ ID NO: 3:

              GCCGAAGCTT  GGATCCGTTT  TAGCTGAATA  AAT                    33
```

## Description

### Field of the Invention

The present invention relates to a novel transgenic plant, more specifically, a herbicide-tolerant transgenic plant transformed with a recombinant expression vector which gives the plant a resistance against diphenylether-derived herbicide.

### Background of the Invention

Phytotoxicity caused by agricultural chemicals, brings about the metabolic disorders in transpiration, assimilation and dissimilation of plants, which finally leads to abnormal growth of the plants. For example, diphenylether(DPE)-derived herbicide which is an ether compound having 2 benzene rings connected by oxygen, has been widely used for the control of weeds, due to its excellent and prolonged weed control efficacy; however, there is a serious problem that it also produces a phytotoxic effect on the crops of interest. Accordingly, there is a need in the art to explore the way of protection of the crops of interest from the agricultural chemicals including DPE-derived herbicide.

On the other hand, protoporphyrinogen oxidase(hereinafter referred to as "Protox") has been identified from yeast, bacteria, mammals and plants(see: Poulson, R. and Polglase, W.J., J. Biol. Chem., 250:1269-1274(1975); Poulson, R. et al., FEBS Lett., 62:351-353(1976); Poulson, R., J. Biol. Chem., 251:3730-3733(1976); Jacobs, J.M. and Jacobs, N.J., Biochem. J., 244:219-224(1987)).

Recently, it has been reported that: Protox in the plastid of plant cell(hereinafter referred to as "P-Protox") is involved in the conversion of protoporphyrinogen to protoporphyrin IX and its activity is inhibited by an agricultural chemical, DPE-derived herbicide(see: Matringe, M. et al., Biochem. J., 260:231-235(1989a); Matringe, M. et al., FEBS Lett., 245:35-48(1989b); Witkowski, D.A. and Halling, B.P., Plant Physiol., 90:1239-1242(1989); Jacobs, J.M. et al., Arch. Biochem. Biophys., 280:369-375(1990); Camadro, J.M. et al., Biochem. J., 267:17-21(1991)); and, therefore, application of the DPE-derived herbicide leads to accumulation of protoporphyrinogen which moves into out of plastid, i.e., cytosol and is oxidized to protoporphyrin IX(see: Jacobs, J.M. and Jacobs, N.J., Plant Physiol., 101:1181-1188(1993)), which in turn, on exposure to light and oxygen, generates singlet oxygen destroying cell membrane and results in cell death and disability(see: Witkowski, D.A. and Halling, B.P., Plant Physiol., 87:632-637(1988); Becerril, J.M. and Duke, S.O., Plant Physiol., 90: 1175-1181(1989)).

On the other hand, it has been also reported that: Protox in plasma membrane of plant cell(hereinafter referred to as "PM-Protox") confers a resistance against DPE-derived herbicide, while it has an activity like P-Protox(see: Lee, H.J. et al., Plant Physiol., 102:881-889(1993)); and, PM-Protox has biochemical properties which is quite different from those of P-Protox, in light of the facts that it is activated by $H_2O_2$ and its activity is repressed by catalase(see: Lee, H.J. and Duke, S.O., J. Agric. Food Chem., 42:2610-2618(1994)). In this connection, it has been known that Protox genes of E. coli K12 and Bacillus subtilis encode proteins of 181 and 470 amino acids, respectively(see: Sasarman, A. et al., Can. J. Microbiol., 39:1155-1161(1993); Hansson, M. and Hederstedt, L., J. Bacteriol., 174:8081-8093(1992)), which have a resistance against DPE-derived herbicide.

Under the circumstances, the present inventors have actively carried out a series of studies on the preparation of transgenic plants which are protected from the phytotoxicity of herbicides; and, they successfully prepared a herbicide-tolerant transgenic tobacco plant which is resistant against a herbicide, DPE-derived herbicide, by employing a process which comprises the step of transforming a recombinant expression vector which contains Protox gene isolated from a genomic library of Bacillus subtilis.

### Summary of the Invention

In accordance with the present invention, it has been discovered that: a transgenic tobacco plant transformed with a recombinant expression vector for Protox is resistant against a herbicide, i.e., DPE-derived herbicide.

A primary object of the invention is, therefore, to provide a herbicide-tolerant transgenic plant transformed with a recombinant vector containing the Protox gene which gives the plant a resistance against DPE-derived herbicide.

The other object of the invention is to provide a process for preparing a DPE-derived herbicide-tolerant transgenic plant.

### Brief Description of Drawings

The above and the other objects and features of the present invention will become apparent from the following description given in conjunction with the accompanying drawings, in which:

Figure 1       is a stepwise construction scheme of expression vector pBP14;

2

Figure 2     is a photograph showing agarose gel electrophoresis pattern of the expression vector pBP14 digested with restriction enzymes;

Figure 3(A)     is a photograph showing shoots induced from the transgenic tobacco cell transformed with expression vector pBP14;

Figure 3(B)     is a photograph showing root generation from the shoots;

Figure 4     is a photograph showing the Northern blot analysis result of mRNA isolated from the transgenic tobacco plant of the invention;

Figure 5     is a photograph showing decolorization patterns of the transgenic tobacco leaves treated with oxyfluorfen; and,

Figure 6     is a graph showing chlorophyll contents of the transgenic tobacco leaves treated with oxyfluorfen.

Detailed Description of the Invention

The present inventors first developed a recombinant expression vector pBP14 containing Protox gene isolated from a genomic library of Bacillus subtilis and made an attempt to prepare a DPE-derived herbicide-tolerant transgenic plant transformed therewith by the aid of a mediator, i.e., Agrobacterium tumefaciens.

To isolate Protox gene conferring a resistance against DPE-derived herbicide, the inventors amplified Protox gene from a genomic library of Bacillus subtilis by employing polymerase chain reaction(hereinafter referred to as "PCR") and fractionated on agarose gel. A 1.4 kb DNA fragment of amplified gene was isolated by unidirectioanl electroelution, digested with HindIII restriction enzyme, and then inserted into HindIII-treated pGEM7Zf(+) to give plasmid pBG14. DNA sequence of 1.4 kb DNA fragment was determined and confirmed as a Protox gene of Bacillus subtilis. pBP14, a recombinant expression vector for Protox, was finally constructed by ligating said 1.4 kb DNA fragment with a binary vector pBl121.

For the expression of isolated Protox gene in plant, Agrobacterium tumefaciens LBA 4404, a well-known mediator for plant cell transformation, was transformed with said pBP14 and transgenic plant cell was prepared by co-cultivation of said organism and leaf disc of tobacco plant. Shoots were induced from the transgenic plant cell on MS selective medium; and, root was generated from said shoots. The plant thus obtained was transferred to soil pot for continuous growth.

Proper insertion of Protox gene to the genome of transgenic tobacco was identified by Southern blot analysis, and its transcription was also verified by employing reverse transcription-PCR method and Northern blot analysis.

Resistance of the transgenic plant against DPE-derived herbicide was examined by observing the degree of retaining inherent color of leaves, which is grounded on the fact that DPE-derived herbicide deprives plant cell of water and color rapidly, and in turn brings about cell death(see: Kenyon W.H. et al., Pestic. Biochem. Physiol., 24:240-250(1985); Kunert, K.J. et al., Rev. Weed Sci., 3:35-56(1987)). When the leaf discs of transgenic tobacco were treated with DPE-derived herbicide and decolorization development thereofs were examined with the naked eye, it was determined that: transgenic tobacco plant was less decolorized than non-transgenic tobacco plant and the degrees of decolorization in the transgenic plants were different from each other, depending on their expression level of Protox.

Further, when the resistance of transgenic plant against DPE-derived herbicide was determined by chlorophyll contents of leaves, it was also determined that transgenic tobacco plants retained chlorophyll much more than non-transgenic tobacco plant. Accordingly, it could be concluded that the transgenic tobacco plants prepared by the invention are resistant against DPE-derived herbicide, grounded on the fact that a recombinant expression vector for Protox made a grant of DPE-derived herbicide resistance to the transgenic plant transformed therewith, by the production of Protox.

The present invention is further illustrated by the following examples, which should not be taken to limit the scope of the invention.

Example 1: Isolation of Protox gene

To isolate Protox gene from a genomic library of Bacillus subtilis, lysate produced from 10 ml of liquid culture($10^{10}$ - $10^{11}$ pfu/ml) of Bacillus subtilis genomic library(Clontech, USA) and 2 ml of chloroform were put into a 50 ml centrifugational tube and centrifuged at 4°C, 8,000 rpm for 10 min. To the supernatant, 50 $\mu$l of $\lambda$-TRAP(Clontech, USA) was added, left under ice-cold condition for 30 min and then centrifuged at 8,000 rpm for 10 min. The pellet was suspended in 1 ml of SM buffer(NaCl 5.8 g/L, $MgSO_4 \cdot 7H_2O$ 2 g/L, 1 M Tris $\cdot$ HCl(pH 7.5) 50 ml/L and 2 % gelatin 5 ml/L), centrifuged at 15,000 rpm for 1 min, and the pellet thus obtained was resuspended in 1 ml of SM buffer, and suspension/centrifugation was repeated twice. The pellet was finally dissolved in 500 $\mu$l of TE-10 buffer(10 mM Tris $\cdot$ HCl(pH 7.6) and 1 mM EDTA(pH 8.0)) and incubated at 70 °C for 5 min, and centrifugation was carried out at 15,000 rpm for 2 min. Phenol/chloroform(1:1, v/v) was added with a ratio of 1:1(v/v) to the supernatant in order to extract phage DNA, and 5 M ammonium acetate and ethanol were treated to precipitate phage DNA.

To amplify Protox gene from the phage DNA prepared together with the creation of restriction sites of HindIII and BamHI at termini of the Protox gene, PCR was carried out by employing 5'-GCCGAAGCTTGGATCCATGAGTGACCGCAAAAA-3'(SEQ ID NO:1) as the N-terminal primer and 5'-GCCGTCTAGAGTTTTAGCTGAATAAAAT-3'(SEQ ID NO:2) as the C-terminal primer. Each cycle of PCR needed denaturation(95 °C, 1 min), annealing(60 °C, 1 min) and extension (72 °C, 1 min), and to effective amplification were 30 cycles required. The amplified DNAs were fractionated on 0.8 % agarose gel, and 1.4 kb of DNA fragment was isolated by unidirectioanl electroelution and then digested with HindIII restriction enzyme. pBG14 was prepared by ligating HindIII-treated pGEM7Zf(+) with 1.4 kb HindIII fragment of Protox gene by $T_4$ DNA ligase.

To confirm whether the 1.4 kb of DNA fragment amplified as above is Protox gene or not, said pBG14 was introduced into E. coli XL1-BLUE and amplification of plasmid followed. E. coli XL1-BLUE was lysed by alkali to isolate Protox gene. Nucleotide sequence of the isolated Protox gene was determined by employing Sequenase(USB, USA), SP6 primer and T7 primer in accordance with Sanger's dideoxy chain termination method(see: Sanger, F. et al., Proc. Natl. Acad. Sci., USA, 74:5463-5467 (1977)). The results confirmed that nucleotide sequence of the Protox gene is identical with that of known in the art(see: Hansson, M., J. Bacteriol., 176:5962(1994)).

Example 2: Preparation of expression vector pBP14

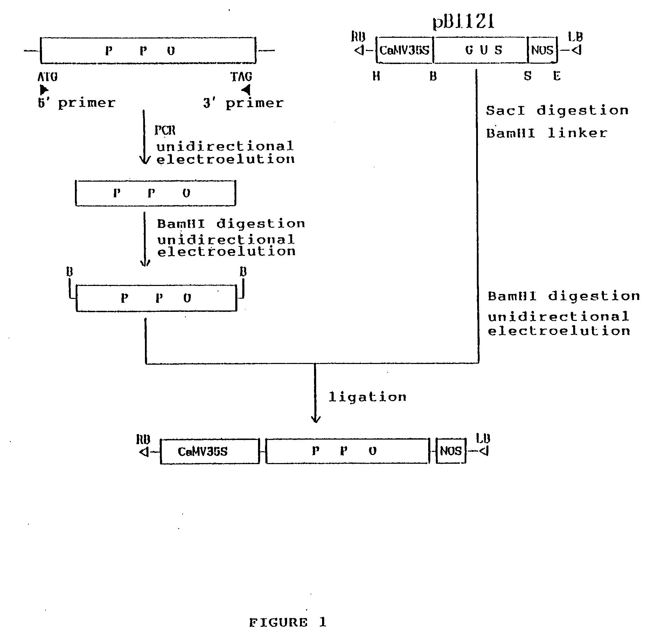

To express the Protox gene prepared in Example 1 under CaMV 35S promoter, about 1.4 kb BamHI fragment of Protox gene was inserted into BamHI-treated binary vector pBI121(Clontech, USA). Plasmid thus produced was named as expression vector pBP14. A construction of pBP14 is schematically illustrated in Fig. 1, where, NOS, CaMV35S, GUS, PPO, B, E, H and S represent terminator of nopaline synthase gene, 35S promoter originated from Cauliflower mosaic virus, gus gene, Protox gene, BamHI, EcoRI, HindIII and SacI restriction sites, respectively.

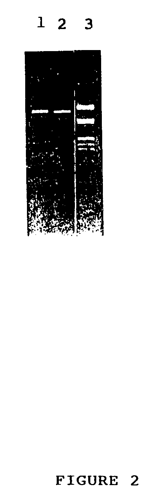

The expression vector pBP14 was introduced into competent E. coli XL1-BLUE treated with $CaCl_2$ and colonies harboring pBP14 were selected. Plasmid was isolated from the colonies thus selected, and digested with BamHI and EcoRI to confirm the proper cloning of Protox gene in forward direction. Fig. 2 shows a gel electrophoresis pattern of pBP14 digested with BamHI or EcoRI on 0.8 %(w/v) agarose gel. In Fig. 2, lanes 1 and 2 represent expression vector pBP14 digested with EcoRI and BamHI, respectively; and, lane 3 represents λDNA digested with HindIII/EcoRI as a molecular marker. As can be seen in Fig. 2, it was clearly demonstrated that the Protox gene was exactly cloned into the expression vector pBP14.

Example 3: Preparation of Agrobacterium mediator for plant cell transformation

Freeze-thawing method was employed to transform Agrobacterium tumefaciens LBA 4404 with pBP14 prepared in Example 2(see: An, G. et al., Plant Molecular Biology Manual, Kluwer Academic Publishers, A3:1-19(1988)). To select Agrobacterium tumefaciens LBA 4404 duly transformed with pBP14, plasmid DNA was isolated from the organism by quick-screening method(see: An, G. et al., Plant Molecular Biology Manual, Kluwer Academic Publishers, A3:1-19(1988)) and digested with BamHI. Agrobacterium tumefaciens LBA 4404 transformed with pBP14 was deposited with an international depository authority(IDA), the Korean Collection of Culture and Microorganism(KCCM) on October 6, 1995 as deposition No. KCCM-10073.

Agrobacterium tumefaciens LBA 4404 transformed with pBP14(KCCM-10073), a mediator for plant cell transformation, was cultured in a shaking incubator at 28°C, 200rpm for 18hrs. After cell culture, said cells were harvested, emulsified with MS medium to the concentration of 1 to 2 x $10^3$ cells/ml and employed for plant cell transformation.

Example 4: Preparation of transgenic tobacco plant

Example 4-1: Transformation of tobacco cell

A tobacco plant, Nicotiana tabacum cv. xanthi, for transformation was prepared as follows: tobacco seed was sterilized with 70 %(v/v) ethanol for 5 min and 50 %(v/v) bleaching agent for 20 min, respectively, washed with distilled water 3 times and incubated on MS basic medium containing 100 mg/l kanamycin and 250 mg/l carbenicillin; and, leaves grown for 1 month in incubator were chopped up in a proper size and employed for plant cell transformation.

Tobacco leaf discs thus obtained were co-cultivated with the Agrobacterium tumefaciens LBA 4404 harboring pBP14 for 30 min and the transformants were incubated on MS medium containing 1.0 mg/l BAP(6-benzylamino purine) and 0.1 mg/l NAA(α-naphthalene acetic acid) at 26°C for 48 hrs under a dark condition.

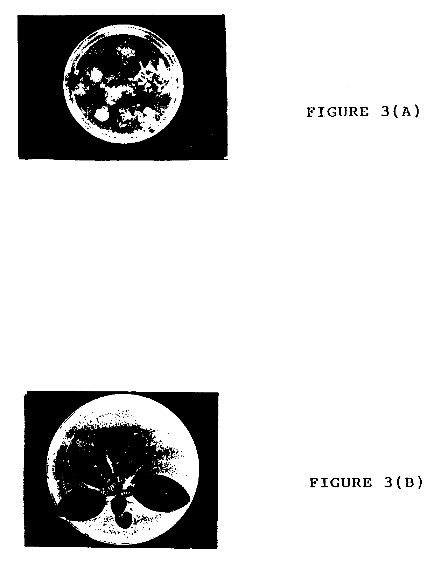

Shoots were induced from the leaf discs, after incubation for 1 week on MS selective medium containing 1.0 mg/l BAP, 100 mg/l kanamycin and 500 mg/l carbenicillin at a temperature of 26°C, with 16 hrs light(about 3000 lux) and 8 hrs dark cycle (see: Fig. 3(A)). The induced shoots were subject to root generation by incubation on MS basic medium, and normal roots were examined 8 days after incubation(see: Fig. 3(B)). The rooted tobacco plants were transferred to soil pots and adapted for later use.

Example 4-2: Identification of Protox gene in transgenic tobacco plant

To determine whether Protox gene was properly inserted to tobacco genome or not, genomic DNA was isolated from the transgenic tobacco plant and PCR was carried out by employing 5'-GCCGAAGCTTGGATCCATGAGTGACCGCAAAAA-3'(SEQ ID NO:1) as the N-terminal primer and 5'-GCCGAAGCTTGGATCCGTTTTAGCTGAATAAAT-3' (SEQ ID NO:3) as the C-terminal primer. Each cycle of PCR needed denaturation(95 $^{\circ}$C, 1 min), annealing(60 $^{\circ}$C, 2 min) and extension(72 $^{\circ}$C, 2 min), and to effective amplification were 40 cycles required. The amplified DNAs were subjected to electrophoresis on 0.8 %(w/v) agarose gel and a band for DNA fragment of 1.4 kb was detected.

To confirm whether said 1.4 kb DNA fragment is the Protox gene or not, the amplified DNAs were also subjected to Southern blot analysis using 1.4 kb HindIII fragment of Example 1 as a probe which was labelled with the DIG-Labelling and Detection Kit (Boehringer Mannheim, Germany). From the Southern blot analysis, it was determined that: the amplified 1.4 kb DNA fragment was Protox gene and it was properly inserted into the genome of transgenic tobacco. Clones 3 and 16 showing large spots in the Southern blot analysis were selected to examine Protox gene expression in the transgenic plants.

Example 4-3: Determination of Protox gene expression in transgenic tobacco plant

To determine whether Protox gene is properly transcribed in the transgenic tobacco plant or not, total cellular mRNAs of the Clones 3 and 16 selected in Example 4-2 and non-transgenic tobacco plant(control) were isolated by employing polyATtract System 1000(Promega, USA) and cDNAs were synthesized therefrom by reverse-transcription PCR using GeneAmp RNA PCR Core Kit(Perkin-Elmer, USA), respectively. From the electrophoresis pattern of the amplified cDNA on 1 %(w/v) agarose gel, a band of 1.4 kb DNA fragment was detected in Clones 3 and 16 which were transformed, whereas it was undetectable in a non-transgenic tobacco plant.

Further, to confirm whether Protox gene was properly expressed in the transgenic tobacco plant or not, Northern blot analysis was carried out. 5 $\mu$g of poly(A)$^+$ RNA was isolated from the transgenic tobacco and electrophoresed on 1 %(w/v) agarose gel containing 10 % formaldehyde. The fractionated mRNAs were transferred to Hybond-N+ Nylon membrane; and, hybridization was followed by employing 1.4 kb HindIII fragment of Protox gene prepared in Example 1 as a probe. In this connection, the probe was prepared by nick translation kit(Promega, USA) and labelled with $\alpha$-$^{32}$P dATP.

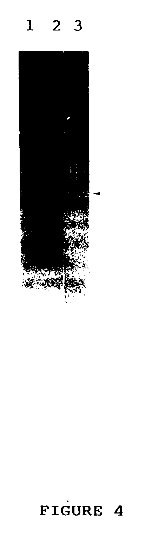

Fig. 4 is a photograph showing the Northern blot analysis of mRNA isolated from the transgenic tobacco plants. In Fig. 4, lane 1 is mRNA from non-transgenic tobacco; lanes 2 and 3 are mRNAs from transgenic tobacco of Clones 3 and 16, respectively; and, arrow represent a transcript. As can be seen in Fig. 4, both of lanes 2 and 3 show a band of transcript at the same position, while no band is showed in non-transgenic tobacco of lane 1; and, mRNA level of Clone 3 is higher than that of Clone 16.

As clearly illustrated in the results of reverse-transcription PCR and Northern blot analysis, it was presumed that the Protox gene was successfully transcribed and expressed in the transgenic tobacco plant of the invention.

Example 4-4: Determination of resistance against oxyfluorfen

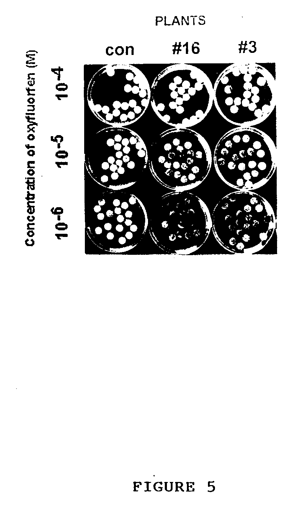

Resistance of transgenic tobacco plant against a DPE-derived herbicide, oxyfluorfen, was determined by examining the degree of decolorization with the naked eye. Leaves under the same growth stage were taken from the transgenic tobacco plant, to cut into a size of 6 mm in diameter. Oxyfluorfen was applied with various concentrations on the surface of leaf discs which were downward, and subjected to a dark condition for 2 hrs. The leaf discs stood for 48 hrs under a light condition and the degree of decolorization was examined with the naked eye(see: Fig. 5).

Fig. 5 is a photograph showing decolorization patterns of the transgenic tobacco leaf discs treated with oxyfluorfen. As shown in Fig. 5, transgenic tobacco leaves were decolorized partially or mildly, while non-transgenic tobacco leaves were decolorized severely at 10$^{-4}$ M, 10$^{-5}$ M and 10$^{-6}$ M of oxyfluorfen. The degree of decolorization was different from each other depending on the expression level of Protox gene in the transgenic plants, which was closely correlated with the results of Northern blot analysis, i.e., leaves of Clone 3 which showed higher expression of Protox were less decolorized than those of Clone 16.

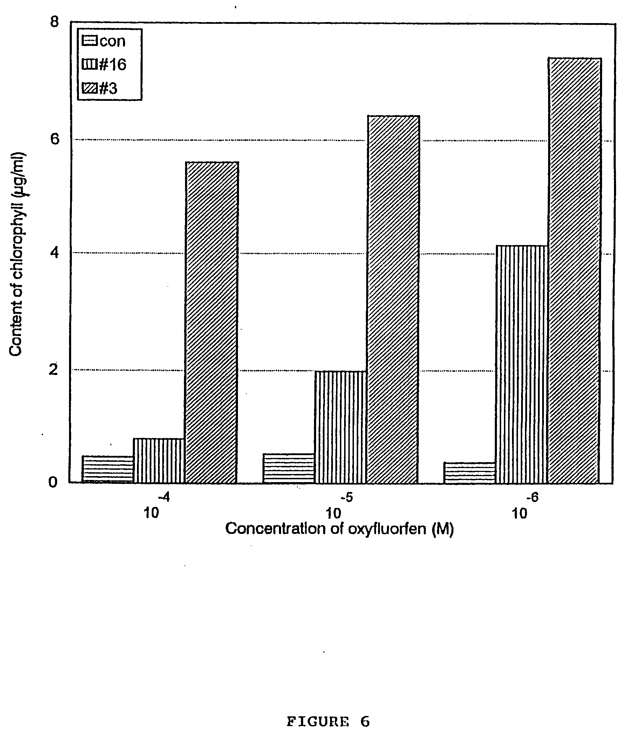

Further, decolorization of the transgenic tobacco plant was also examined by measuring chlorophyll contents of leaves: chlorophyll was extracted with 80 %(v/v) acetone from the leaf discs, and absorbance at 665 nm and 649 nm was observed. Total chlorophyll content was determined by a formula as below and the results were illustrated in the graph of Fig. 6.

$$\text{Total chlorophyll content}(\mu g/ml) = 6.45 \times A_{665} + 17.72 \times A_{649}$$

As can be seen in Fig. 6, transgenic tobacco plants revealed much higher levels of chlorophyll than non-transgenic tobacco(con); and, Clone 3(#3), in particular, showed higher level than that of Clone 16(#16). As demonstrated in the results of examination with the naked eye and measurement of chlorophyll content, it could be concluded that the expression of Protox gene gives a resistance against oxyfluorfen on the tobacco plant transformed with the recombinant expression vector which is capable of expressing Protox.

As clearly illustrated and demonstrated above, the present invention provides a herbicide-tolerant transgenic plant producing protoporphyrinogen oxidase(Protox) which gives the plant a resistance against DPE-derived herbicide, and a process for preparing the same. The process for preparing a herbicide-tolerant transgenic plant comprises the step of culturing plant cells transformed with a recombinant expression vector containing Protox gene. The transgenic plant of the invention possesses a resistance against DPE-derived herbicide with the help of recombinant Protox. Accordingly, the process for preparing the herbicide-tolerant transgenic plant of the invention, can be applied for the preparation of a variety of plants which are protected from the phytotoxicity of DPE-derived herbicide by way of introducing proper recombinant expression vectors containing Protox gene.

**Claims**

1.  A herbicide-tolerant transgenic plant transformed with a recombinant expression vector containing Protox (protoporphyrinogen oxidase) gene which gives the plant a resistance against DPE(diphenylether)-derived herbicide.

2.  The herbicide-tolerant transgenic plant of claim 1, wherein the recombinant expression vector is pBP14 which is capable of expressing Protox(protoporphyrinogen oxidase).

3.  The herbicide-tolerant transgenic plant of claim 1, wherein the Protox(protoporphyrinogen oxidase) gene is originated from Bacillus subtilis.

4.  The herbicide-tolerant transgenic plant of claim 1, wherein the plant is tobacco.

5.  A process for preparing a herbicide-tolerant transgenic plant, which comprises the step of culturing plant cells transformed with a recombinant expression vector containing Protox(protoporphyrinogen oxidase) gene which gives the plant a resistance against DPE(diphenylether)-derived herbicide.

6.  The process of claim 5, wherein the plant cells are transformed with the recombinant expression vector by the aid of Agrobacterium tumefaciens LBA 4404 transformed with a recombinant expression vector pBP14(KCCM-10073).

FIGURE 1

1 2 3

FIGURE 2

FIGURE 3(A)

FIGURE 3(B)

1  2  3

FIGURE  4

FIGURE 5

FIGURE 6